# EUROPEAN PATENT SPECIFICATION

(11) **EP 1 946 763 B1**
(45) Date of publication and mention of the grant of the patent: **21.08.2013**
(21) Application number: 06746949.4
(22) Date of filing: 29.05.2006
(51) Int. Cl.: A61K 33/10, A61K 9/08, A61K 47/04, A61K 47/26, A61K 47/18, A61P 25/02, A61P 25/26, A61K 47/02, A61K 9/00, A61K 31/195, A61K 31/7004, A61K 33/00

(54) **PROMOTER FOR RECOVERY FROM ANESTHESIA**
MITTEL ZUR FÖRDERUNG DER ERHOLUNG NACH EINER NARKOSE
AGENT FACILITANT LE REVEIL APRES UNE ANESTHESIE

(30) Priority: 08.11.2005 JP 2005323968
(43) Date of publication of application: 23.07.2008
(73) Proprietor: Ajinomoto Co., Inc., Tokyo 104-8315 (JP)
(72) Inventor: SATOU, Kazunori, Shizuoka-shi, Shizuoka 4240911 (JP); OGAWA, Takashi, Shizuoka-shi, Shizuoka 4240911 (JP); KOUYAMA, Tetsuya, Kawasaki-shi, Kanagawa 2100801 (JP); MORI, Saori, Shizuoka-shi, Shizuoka 4240911 (JP)
(74) Representative: Nicholls, Kathryn Margaret
(86) International application number: PCT/JP2006/310671
(87) International publication number: WO 2007/055044

(56) References cited:
- EP-A1- 1 447 086
- WO-A1-86/00227
- JP-A- 10 203 961
- JP-A- 2004 149 495
- US-A- 5 723 281
- US-A1- 2002 064 566
- US-A1- 2003 180 386
- US-A1- 2004 175 437
- US-A1- 2005 100 615
- US-B1- 6 328 997
- ROTH S.H. ET AL: 'Cellular mechanisms of neural excitation induced by general anesthetics' PROGRESS IN ANESTHETIC MECHANISM vol. 6, 2000, pages 63 - 67, XP003012630

## Description

### TECHNICAL FIELD

The present invention relates to a composition comprising bicarbonate ions, for use in a method of anesthesia arousal that facilitates awakening from anesthesia. In particular, the present invention relates to an anesthesia arousal composition that facilitates awakening from perioperative anesthesia. The present invention further relates to a method for controlling and facilitating awakening from anesthesia by administering the anesthesia arousal composition.
The present invention also relates to an acidosis-correcting composition, as well as to a method for maintaining near-normal blood pH and controlling and facilitating awakening from anesthesia.

### BACKGROUND ART

In surgical operations, patients are put under anesthesia. In other words, patients are anesthetized during the perioperative period. There are two general types of surgical anesthesia: general anesthesia and regional anesthesia.

General anesthesia is performed during relatively large, complicated surgeries and includes inhalation anesthesia (gas anesthesia) and intravenous anesthesia. Inhalation anesthesia uses inhalation anesthetics such as ether, halothane, enflurane, isoflurane, methoxyflurane and seveflurane. Inhalation anesthetics are generally volatile and have the advantage of being absorbed and discarded via the lungs. These agents also have a common characteristic of prompt induction and awakening from anesthesia. Nonetheless, inhalation anesthesia (gas anesthesia) has a serious effect (side effect) of suppressing the function of the respiratory and cardiovascular systems.

Intravenous anesthesia uses intravenous anesthetics such as pentobarbital, thiopental, methohexital and propofol. Once intravenously injected, intravenous anesthetics quickly reach the target organ (brain) and produce unconsciousness. Intravenous anesthetics are divided into different types, such as short-acting and long-acting, depending on these mechanisms. The drug currently most widely used in Japan is propofol, an intravenous anesthetic for long-term use. Commercially available products of propofol include 1% Diprivan injection (AstraZeneca) and 1% propofol injection (Maruishi Pharmaceutical Co., Ltd.).

Although inhalation anesthesia has long been the major anesthetic technique used in Japan, it is less frequently used now as the long-acting intravenous anesthetics have become available. Inhalation anesthesia and intravenous anesthesia are often used in combination.

Since maintenance and awakening from general anesthesia provided during large complicated surgeries are difficult to control, such surgeries are generally performed by a team of surgeons and experienced anesthesiologists who control the condition of the patient under anesthesia.

General anesthesia acts on the central nervous system (brain) by reducing the level of its activity. The decreased activity of the brain, an organ that plays a central role in the control of the systemic metabolism, leads to decreased metabolism of the body. This implies that delayed awakening from anesthesia delays the recovery of the body's metabolism, which in turn delays the recovery of spontaneous respiration and keeps the activity of tissues and organs low. As a result, the action of biological defense may be delayed or the immune activity may be decreased, resulting in an increased risk of complication.

For this reason, early awakening from anesthesia is desired once surgery under general anesthesia has been finished. Given the fact that considerable time and labor are spent on the care of patients in intensive care units (ICUs) when the awakening of the patients from anesthesia is delayed, early awakening from anesthesia is crucial in not only reducing the workload of medical staff, but also in ensuring effective postoperative recovery of the patients.

However, the only way to accelerate awakening from anesthesia is to control anesthesia by varying the administration rate based on how the operation proceeds and how the depth of anesthesia (condition of the body) changes correspondingly over the course of the operation. This can be done only by experiences of anesthesiologists.
Patent Document 1: Japanese Laid-Open Patent Publication No. 2004-149495

### DISCLOSURE OF THE INVENTION

### PROBLEMS TO BE SOLVED BY THE INVENTION

In view of the foregoing problems, it is an object of the present invention to provide a method for facilitating postoperative awakening from anesthesia that does not depend on the experience of anesthesiologists. It is another object of the present invention to provide an anesthesia arousal composition for use in the method.

In the course of our study to achieve the above-described objects, the present inventors have found that awakening from anesthesia is facilitated in perioperative (intraoperative) patients administered a perioperative infusion fluid containing bicarbonate ion, as compared to those administered a perioperative infusion fluid containing sodium acetate sodium acetate or sodium lactate.

### MEANS FOR SOLVING THE PROBLEMS

Thus, the present invention in one aspect concerns the following:
(1) An anesthesia arousal composition containing a bicarbonate ion;
(2) The anesthesia arousal composition according to (1) above, wherein the bicarbonate ion is contained as an electrolyte; and
(3) The anesthesia arousal composition according to (1) or (2) above, containing sodium bicarbonate as a major component that serves as a source of the bicarbonate ion, along with each or a combination of another electrolyte, glucose and an amino acid.
   More specifically, the present invention concerns the following:
(4) The anesthesia arousal composition according to (1), (2) or (3) above, provided in the form of Ringer's solution.

The present invention in another aspect concerns the following:
(5) An acidosis-correcting composition containing a bicarbonate ion;
(6) The acidosis-correcting composition according to (5) above, wherein the bicarbonate ion is contained as an electrolyte; and
(7) The acidosis-correcting composition according to (5) or (6) above, containing sodium bicarbonate as a major component that serves as a source of the bicarbonate ion, along with each or a combination of another electrolyte, glucose and an amino acid.
   More specifically, the present invention concerns the following:
(8) The acidosis-correcting composition according to (5), (6) or (7) above, provided in the form of Ringer's solution.

The present invention in still another aspect concerns the following:
(9) A method for controlling and facilitating awakening from anesthesia, comprising administering to a perioperative anesthetized patient the anesthesia arousal composition or the acidosis-correcting composition according to any of (1) to (8) above.

### EFFECT OF THE INVENTION

The anesthesia arousal composition provided by the present invention is essentially an infusion fluid containing a bicarbonate ion. More specifically, it is an infusion fluid that contains sodium bicarbonate as a major component that serves as a source of the bicarbonate ion (electrolyte). The anesthesia arousal composition facilitates early postoperative awakening from general anesthesia.
The infusion fluid of the present invention, containing sodium bicarbonate as a major component that serves as a source of the bicarbonate ion (electrolyte), increases protein binding of administered anesthetics by immediately correcting acidosis and maintaining normal or near-normal blood pH. In this manner, the infusion fluid can facilitate early postoperative awakening of patients from general anesthesia.

If patients can awaken from anesthesia fast enough, the recovery of the systemic metabolism is not delayed, so that spontaneous respiration recovers quickly and tissues and organs resume their normal function quickly. As a result, increased risk of complication caused by the delayed action of biological defense or decreased immune activity can be avoided.

In addition, the care required by patients in ICU because of delayed awakening can be reduced and, as a result, the workload of medical staff can be decreased.
Furthermore, postoperative awakening from general anesthesia, a procedure that could be performed only by experienced anesthesiologists, is done by simple administration of the bicarbonated-Ringer's solution. Thus, the present invention enables stable, early awakening from general anesthesia that does not depend on the experience of anesthesiologists.

### BRIEF DESCRIPTION OF THE DRAWINGS

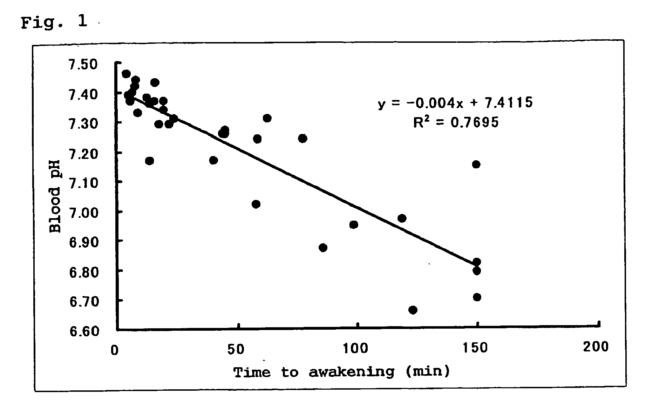

Fig. 1 is a diagram showing the results of Example 6.

### BEST MODE FOR CARRYING OUT THE INVENTION

As described above, the anesthesia arousal or the acidosis-correcting composition provided by the present invention (which is referred to simply as "anesthesia arousal composition," hereinafter) contains a bicarbonate ion that acts to facilitate awakening from anesthesia. More specifically, it is a preparation, preferably an infusion fluid, which contains sodium bicarbonate, a major component that serves as a source of the bicarbonate ion (electrolyte), along with each or a combination of another electrolyte, glucose and an amino acid. The anesthesia arousal composition is provided in the form of a Ringer's solution, a maintenance solution, a starting solution, a solution for correction of dehydration, or a solution for postoperative recovery, in particular in the form of a Ringer's solution.

When the anesthesia arousal composition of the present invention containing a bicarbonate ion is provided in the form of a Ringer's solution, a type of infusion fluid used to replace the extracellular fluid, it contains a bicarbonate ion preferably at a concentration of 20 to 40 mEq/L and more preferably at a concentration of 22 to 30 mEq/L. Preferably, it also contains other electrolytes: 130 to 145 mEq/L of sodium ion; 2 to 5 mEq/L of potassium ion; 90 to 130 mEq/L of chlorine ion; 2 to 5 mEq/L of calcium ion; 0.5 to 2.5 mEq/L of magnesium ion; and 0 to 7 mEq/L citrate ion, along with 0 to 5 g/L of glucose.

When the anesthesia arousal composition of the present invention containing a bicarbonate ion is provided in the form of a maintenance solution, another type of infusion fluid, it contains a bicarbonate ion preferably at a concentration of 15 to 30 mEq/L and more preferably at a concentration of 18 to 25 mEq/L. Preferably, it also contains other electrolytes: 30 to 40 mEq/L of sodium ion; 15 to 25 mEq/L of potassium ion; and 30 to 40 mEq/L of chlorine ion, along with 40 to 80g/L of glucose.

When the anesthesia arousal composition of the present invention containing a bicarbonate ion is provided in the form of a starting solution, a solution for correction of dehydration, or a solution for postoperative recovery, it contains a bicarbonate ion and electrolytes at concentrations suitable for its intended use.

Specifically, when the composition is intended as a starting solution, it preferably contains 30 to 90 mEq/L of sodium ion, 35 to 80 mEq/L of chlorine ion, 20 to 30 mEq/L of bicarbonate ion and 25 to 40 g/L of glucose. When it is intended as a solution for correction of dehydration, it preferably contains 60 to 90 mEq/L of sodium ion, 20 to 30 mEq/L of potassium ion, 0 to 5 mEq/L of magnesium ion, 45 to 70 mEq/L of chlorine ion, 5 to 10 mmol/L of phosphorus, 20 to 50 mEq/L of bicarbonate ion and 10 to 35 g/L of glucose. When it is intended as a solution for postoperative recovery, it preferably contains 30 mEq/L of sodium ion, 5 to 10 mEq/L of potassium ion, 20 to 30 mEq/L of chlorine ion, 10 to 20 mEq/L of bicarbonate ion and 30 to 50 g/L of glucose.

Any electrolytes may be used suitable for the intended use. Examples thereof include sodium chloride, sodium citrate, sodium acetate, sodium lactate, sodium dihydrogen phosphate, disodium hydrogen phosphate, sodium gluconate, sodium glycerophosphate, sodium malate, potassium chloride, dibasic potassium phosphate, potassium acetate, potassium citrate, potassium lactate, potassium glycerophosphate, potassium malate, calcium chloride, calcium lactate, calcium gluconate, calcium glycerophosphate, dibasic calcium phosphate, calcium malate, magnesium chloride, magnesium gluconate and magnesium glycerophosphate.

Of these components, sodium chloride, potassium chloride, calcium chloride, magnesium chloride, sodium bicarbonate, sodium citrate and glucose are particularly preferred.

The anesthesia arousal composition of the present invention containing a bicarbonate ion is intended for use as an infusion fluid. However, a stable preparation containing sodium bicarbonate ion is difficult to prepare since sodium bicarbonate that serves as a source of bicarbonate ion, an important base required to maintain the acid-base equilibrium of extracellular fluid, tends to react with calcium and magnesium to form insoluble calcium carbonate and magnesium carbonate, and since an aqueous sodium bicarbonate solution, when left or heated, produces carbon dioxide that increases the pH of the solution. For this reason, the infusion fluid of the present invention containing a bicarbonate ion may be prepared either upon use, or as separate solutions of sodium bicarbonate and an electrolyte, which may be contained in a two separate chambers of a container. For convenience upon use, a single solution-type preparation is preferred.

When used as an infusion fluid, the anesthesia arousal composition of the present invention containing a bicarbonate ion is stable and can be administered to perioperative patients to facilitate their early awakening from general anesthesia. The patients administered the infusion fluid of the present invention awaken from general anesthesia earlier than the patients administered other infusion fluids containing sodium acetate or sodium lactate.

In a storage stability test, the infusion fluid of the present invention did not undergo any observable changes during the storage period despite the presence of a certain concentration of carbon dioxide in the space of the container. The infusion fluid remained stable without its components decomposed or forming precipitation.

When used as an infusion fluid, the anesthesia arousal composition of the present invention containing a bicarbonate ion is preferably administered to perioperative anesthetized patients (perioperative infusion) to facilitate their awakening from anesthesia. In particular, when the composition is prepared as a Ringer's solution, one of perioperative infusion fluids, it is used in the following manner.
Specifically, it is administered during surgery to a patient under general anesthesia to replace the extracellular fluid in blood. The replacing fluid is also properly administered following the surgery to facilitate early awakening of the patient from general anesthesia.

Whether in this or other forms, the anesthesia arousal composition of the present invention can be administered to perioperative anesthetized patients to control/facilitate their awakening from anesthesia.

The studies conducted by the present applicants have demonstrated that the blood pH is correlated with the time required for awakening from anesthesia. This means that: the lower the pH, the longer it takes for the patient to awaken from anesthesia.
In fact, one study conducted to examine the protein binding of propofol to human serum albumin showed that the protein binding of propofol decreased as the pH was decreased.
This observation suggests that since more propofol exists in its protein-unbound form at a lower pH, the anesthetic effect of propofol is enhanced at such a low pH.
Thus, by increasing the blood pH from a lower value, the protein binding of the anesthetic can be increased and, as a result, awakening from anesthesia can be accelerated.
Since the anesthesia arousal composition of the present invention can effectively correct acidosis, it can maintain normal or near-normal blood pH and can thereby accelerate awakening of patients from anesthesia.

When used as a Ringer's solution in partially hepatectomized rat model, the anesthesia arousal composition of the present invention containing a bicarbonate ion significantly accelerated awakening from anesthesia as compared to lactated-Ringer's solution. It also significantly accelerated awakening in the STZ-induced diabetic ketoacidosis model as compared to acetated-Ringer's solution. Unlike sodium acetate or sodium lactate, sodium bicarbonate produce bicarbonate ion without any intervening metabolic process. Thus, it can be used as an alkalizer in patients with metabolic disorder or organ dysfunction, and maintain a higher blood pH as compared with other Ringer's solutions. Accordingly, the anesthesia arousal composition of the present invention can be administered to perioperative (intraoperative) patients to facilitate their awakening from anesthesia.

### EXAMPLES

The present invention will now be described with reference to examples.

### Example 1: Storage stability

Ringer's solutions containing 20.0, 22.5, 25.0, 27.5 and 30.0 mEq/L of a bicarbonate ion (HCO₃⁻) were prepared.

Specifically, the infusion preparations were prepared according to the formulations shown in Table 1 below. For each preparation, the components were dissolved in water to make a 10L solution (measured pH = 8.0). Carbon dioxide was bubbled through the solution to adjust the pH to 6.5. The solution was then filtered and loaded in a 500 mL glass vial. The vial was autoclaved at 115°C for 15 min. In this manner, five different Ringer's solutions containing 20.0, 22.5, 25.0, 27.5 and 30.0 mEq/L of a bicarbonate ion (HCO₃⁻) were prepared.

**(Table 1)**

| Components (g) | Bicarbonate ion concentration (mEq/L) | | | | |
|---|---|---|---|---|---|
| | 20.0 | 22.5 | 25.0 | 27.5 | 30.0 |
| Sodium chloride | 64.3 | 62.8 | 61.4 | 59.9 | 58.4 |
| Potassium chloride | 2.98 | 2.98 | 2.98 | 2.98 | 2.98 |
| Calcium chloride dihydrate | 2.21 | 2.21 | 2.21 | 2.21 | 2.21 |
| Magnesium chloride hexahydrate | 1.02 | 1.02 | 1.02 | 1.02 | 1.02 |
| Sodium bicarbonate | 16.8 | 18.9 | 21.0 | 23.1 | 25.2 |
| Sodium citrate dihydrate | 4.90 | 4.90 | 4.90 | 4.90 | 4.90 |

At the beginning and after a three-month storage period at room temperature, the infusion fluids (Ringer's solutions) were analyzed for pH, insoluble material, insoluble particle count, amounts of components and carbon dioxide concentration in the vial space. The results are shown in Tables 2 and 3 below. As can be seen from the results, each of the Ringer's solutions of the present invention did not undergo any significant changes during the storage period: each solution proved to be a stable infusion fluid that did not decompose or form precipitation during the storage period.

**(Table 2)**

| | | | Bicarbonate ion concentration (mEq/L) | | | | | |
|---|---|---|---|---|---|---|---|---|
| | | | 20.0 | | 22.5 | | 25.0 | |
| | | | Initial | 3M | Initial | 3M | Initial | 3M |
| pH | | | 7.2 | 7.1 | 7.1 | 7.2 | 7.1 | 7.1 |
| Insoluble material test | | | ND | ND | ND | ND | ND | ND |
| Insoluble particles (particles/mL or less) | | 10 µM > | 0.0 | 0.5 | 0.1 | 0.9 | 0.0 | 0.4 |
| | | 22 µM > | 0.0 | 0.0 | 0.0 | 0.0 | 0.0 | 0.0 |
| Contents (w/v%) | Na | | 0.302 | 0.302 | 0.304 | 0.303 | 0.303 | 0.303 |
| | K | | 0.015 | 0.015 | 0.015 | 0.015 | 0.015 | 0.015 |
| | Ca | | 0.00582 | 0.00584 | 0.00582 | 0.00586 | 0.00583 | 0.00587 |
| | Mg | | 0.00114 | 0.00115 | 0.00116 | 0.00111 | 0.00116 | 0.00112 |
| | Chlorine | | 0.4168 | 0.4144 | 0.4081 | 0.4065 | 0.4001 | 0.3974 |
| | Bicarbonate | | 0.119 | 0.116 | 0.134 | 0.132 | 0.149 | 0.149 |
| | Citric acid | | 0.0309 | 0.0318 | 0.0310 | 0.0319 | 0.0309 | 0.0320 |
| Space (CO₂%) | | | 7.16 | 9.85 | 5.40 | 9.53 | 6.10 | 10.24 |

| | | | | | | | | |
|---|---|---|---|---|---|---|---|---|
| ND: Not detected | | | | | | | | |

**(Table 3)**

| | | | Bicarbonate ion concentration (mEq/L) | | | |
|---|---|---|---|---|---|---|
| | | | 27.5 | | 30.0 | |
| | | | Initial | 3M | Initial | 3M |
| pH | | | 7.2 | 7.1 | 7.1 | 7.2 |
| Insoluble material test | | | ND | ND | ND | ND |
| Insoluble particles (particles/mL or less) | | 10 µM > | 0.0 | 0.2 | 0.0 | 0.1 |
| | | 22 µM > | 0.0 | 0.0 | 0.0 | 0.0 |
| Contents (w/v%) | Na | | 0.302 | 0.305 | 0.304 | 0.306 |
| | K | | 0.015 | 0.015 | 0.015 | 0.015 |
| | Ca | | 0.00586 | 0.00589 | 0.00594 | 0.00592 |
| | Mg | | 0.00116 | 0.00113 | 0.00115 | 0.00114 |
| | Chlorine | | 0.3932 | 0.3984 | 0.3865 | 0.3830 |
| | Bicarbonate | | 0.163 | 0.165 | 0.183 | 0.178 |
| | Citric acid | | 0.0310 | 0.0322 | 0.0312 | 0.0322 |
| Space (CO₂%) | | | 8.02 | 10.42 | 10.14 | 11.90 |

| | | | | | | |
|---|---|---|---|---|---|---|
| ND: Not detected | | | | | | |

### Example 2: Awakening time in the rat model (partial hepatectomy, short surgery)

### [Method]

A Ringer' solution (anesthesia arousal composition of the present invention containing a bicarbonate ion) was prepared according to the formulation shown in Table 4 below. The solution was administered to 7-week-old male SD rats via a catheter inserted into the right jugular vein at a rate of 20 mL/kg/hr. Starting 30 minutes after the beginning of administration of the Ringer's solution, administration of propofol, an intravenous anesthetic, was started at a rate of 45 mg/kg/hr. As the anesthetic was started, the abdomen was cut open and about 75% of the liver (each of the left and right outer lobes and the left inner lobe) was removed after 15 minutes. 30 minutes after starting surgery, the abdomen was closed and the surgery and the administration of the anesthetic were finished. The administration of the Ringer's solution was continued until 30 minutes after the termination of the anesthetic (total of 90 minutes).
The time required for awakening from anesthesia was measured and compared among the groups. The awakening of a rat was determined as the time when it regained the righting reflex and resumed walking.

As controls, an acetated-Ringer's solution and a lactated-Ringer's solution were prepared according to the formulations shown in Table 4 below and administered in the same manner. The tested groups were as follows:
the group administered the Ringer's solution of the present invention = 29 cases;
the group administered the acetated-Ringer's solution = 10 cases; and
the group administered the lactated-Ringer's solution = 19 cases.

**(Table 4)**

| Components | Formulation (g/500mL) | | |
|---|---|---|---|
| | Ringer's solution of the present invention | Acetated-Ringer's solution | Lactated-Ringer's solution |
| Sodium bicarbonate | 1.05 | - | |
| Sodium acetate | - | 1.90 | - |
| Sodium lactate | - | - | 1.55 |
| Sodium chloride | 3.07 | 3.00 | 3.00 |
| Potassium chloride | 0.15 | 0.15 | 0.15 |
| Magnesium chloride | 0.051 | - | - |
| Calcium chloride | - | 0.10 | 0.10 |
| Sodium citrate | 0.245 | - | - |

### [Results]

The time it took for the animals of each group to awaken from anesthesia was shown in Table 5.

**(Table 5)**

| Groups | Time to awakening (min) |
|---|---|
| Ringer's solution of the present invention | 25.2± 9.8 |
| Acetated-Ringer's solution | 40.7±27.6 |
| Lactated-Ringer's solution | 39.2±16.0 |

The results indicate that the time to awakening is significantly shorter in the group administered the Ringer's solution of the present invention (anesthesia arousal composition) as an infusion fluid than in the group administered the lactated-Ringer's solution (p < 0.05). The awakening time in the group administered the Ringer's solution of the present invention is also relatively shorter than that in the group administered the acetated-Ringer's solution (p < 0.06).
These results clearly demonstrate the ability of the anesthesia arousal composition of the present invention to facilitate awakening from anesthesia.

### Example 3: Awakening rate in the rat model (partial hepatectomy, long surgery)

### [Method]

A Ringer's solution (anesthesia arousal composition containing a bicarbonate ion of the present invention) was prepared according to the formulation shown in Table 4 above. The solution was administered to 7-week-old male SD rats via a catheter inserted into the right jugular vein at a rate of 20 mL/kg/hr. At the same time, propofol, an intravenous anesthetic, was started at a rate of 45 mg/kg/hr. As the administration was started, the abdomen was cut open and about 75% of the liver (each of the left and right outer lobes and the left inner lobe) was removed after 30 minutes. 60 minutes after starting surgery, the abdomen was closed and the surgery was finished. The administration of the Ringer's solution and the anesthetic was continued until 30 minutes after the completion of the surgery (total of 90 minutes). The time required for awakening from anesthesia was measured and compared among the groups. The awakening of a rat was determined as the time when it regained the righting reflex and resumed walking.

As controls, an acetated-Ringer's solution and a lactated-Ringer's solution were prepared according to the formulations shown in Table 4 above and administered in the same manner. The tested groups were as follows:
the group administered the Ringer's solution of the present invention = 38 cases;
the group administered the acetated-Ringer's solution = 20 cases; and
the group administered the lactated-Ringer's solution = 18 cases.

### [Results]

For each group, the ratio of arousal state and the average awakening time observed 2, 3 and 4 hours after the termination of the anesthetic were shown in Table 6 below.

**(Table 6)**

| | % awakening | | | Average awakening time (min) |
|---|---|---|---|---|
| | 2 hours | 3 hours | 4 hours | |
| Ringer's solution of the present invention | 10% | 50% | 70% | 190.6 ± 50.7 |
| Acetated-Ringer's solution | 5% | 10% | 50% | 215.2 ± 32.2 |
| Lactated-Ringer's solution | 0% | 11% | 33% | 219.9 ± 32.4 |

As can be seen from the results, the awakening rate at each time point up to 4 hours after the termination of anesthetic tends to be higher in the group administered the Ringer's solution of the present invention (anesthesia arousal composition) as an infusion fluid than in the groups administered the lactated-Ringer's solution or the acetated-Ringer's solution.
The average awakening time was shorter in the group administered the Ringer's solution of the present invention than in the group administered the lactated-Ringer's solution (p < 0.05).
These results clearly demonstrate the ability of the anesthesia arousal composition of the present invention to facilitate awakening from anesthesia.

### Example 4: Change in the blood anesthetic levels in the rat model (partial hepatectomy, short surgery)

### [Method]

A Ringer' solution (anesthesia arousal composition containing a bicarbonate ion of the present invention) was prepared according to the formulation shown in Table 4 above. The solution was administered to groups of 7 male SD rats, fasted for about 16 hours before the test, via a catheter placed in the central vein at a rate of 20 mL/Kg/hr. Starting 30 minutes after the beginning of administration of the Ringer's solution, propofol (an intravenous anesthetic, 1% Diprivan injection) was administered at a rate of 45 mg/kg/hr. At the same time, the abdomen was cut open and about 75% of the liver was excised 15 minutes after the beginning of the anesthetic. The abdomen was closed 30 minutes after the starting surgery. The anesthetic was continued for the 30-minute period of surgery. The Ringer's solution was continued until 30 minutes after the termination of the anesthetic (total of 90 minutes).
Blood samples were collected 15 minutes after the beginning of the anesthetic, immediately before termination of the anesthetic, and 5, 30, 60 and 90 minutes after the termination of the anesthetic. The collected samples were centrifuged and the plasma was analyzed for the concentration of the anesthetic.
As controls, an acetated-Ringer's solution and a lactated-Ringer's solution were prepared according to the formulations shown in Table 4 above and administered in the same manner.

### [Results]

The changes in the plasma concentration of the anesthetic (propofol) were shown in Table 7 (Ringer's solution of the present invention), Table 8 (acetated-Ringer's solution) and Table 9 (lactated-Ringer's solution) below.

**(Table 7)**

| Ringer's solution of the present invention | | | | | | | | | |
|---|---|---|---|---|---|---|---|---|---|
| Time (min) | Plasma anesthetic level in rats (ng/mL) | | | | | | | Mean (ng/mL) | |
| | 1 | 2 | 3 | 4 | 5 | 6 | 7 | | SD |
| -15 | 2466.5 | 1911.2 | 2931.6 | 1807.7 | 2229.0 | 2395.5 | 1234.7 | 2139.5 | 546.0 |
| 0 | 3487.1 | 2350.0 | 4110.3 | 2288.4 | 3237.4 | 2754.4 | 3787.5 | 3145.0 | 705.8 |
| 5 | 2701.6 | 1615.5 | 2339.0 | 1901.5 | 1903.5 | 2123.1 | 2475.4 | 2149.9 | 376.6 |
| 30 | 1143.2 | 984.5 | 1198.1 | 1031.3 | 1224.9 | 1269.2 | 1185.9 | 1148.2 | 104.0 |
| 60 | 933.7 | 648.2 | - | 606.9 | 704.1 | 683.7 | 676.8 | 710.6 | 114.2 |
| 90 | 835.6 | 603.5 | - | 719.3 | 109.3 | 153.0 | 642.5 | 610.5 | 237.8 |

**(Table 8)**

| Acetated-Ringer's solution | | | | | | | | | |
|---|---|---|---|---|---|---|---|---|---|
| Time (min) | Plasma anesthetic level in rats (ng/mL) | | | | | | | Mean (ng/mL) | |
| | 1 | 2 | 3 | 4 | 5 | 6 | 7 | | SD |
| -15 | 2300.1 | 3517.3 | 2092.0 | 2120.2 | 1647.6 | 1975.1 | 1797.9 | 2207.2 | 616.5 |
| 0 | 3046.0 | 4639.2 | 2831.9 | 3347.5 | 2707.2 | 2666.9 | 2928.1 | 3166.7 | 688.7 |
| 5 | 2140.4 | 2441.4 | 1802.9 | 2348.0 | 1921.8 | 2056.0 | 1745.4 | 2065.1 | 264.2 |
| 30 | 1147.6 | 1141.4 | 742.4 | 1066.2 | 991.4 | 722.3 | 701.2 | 930.4 | 202.1 |
| 60 | 431.2 | 809.4 | - | 470.7 | 602.4 | 448.8 | 392.0 | 525.8 | 156.3 |
| 90 | - | 761.3 | - | 3495.6 | 494.3 | 423.4 | 394.1 | 513.7 | 145.3 |

**(Table 9)**

| Lactated-Ringer's solution | | | | | | | | | |
|---|---|---|---|---|---|---|---|---|---|
| Time (min) | Plasma anesthetic level in rats (ng/mL) | | | | | | | Mean (ng/mL) | |
| | 1 | 2 | 3 | 4 | 5 | 6 | 7 | | SD |
| -15 | 2582.7 | 2715.1 | 1857.7 | 2000.6 | 3722.9 | 2080.5 | 2997.1 | 2565.2 | 658.8 |
| 0 | 2626.6 | 3890.9 | 2602.2 | 3274.3 | 4652.8 | 3176.5 | 4443.7 | 3523.9 | 825.9 |
| 5 | 2104.5 | 2671.2 | 1662.8 | 1373.5 | 2999.5 | 1973.7 | 3387.0 | 2310.3 | 732.2 |
| 30 | 985.9 | 1837.8 | 819.5 | 155.5 | 1163.8 | 1044.7 | 1867.5 | 1125.0 | 594.4 |
| 60 | 646.4 | 733.0 | 1353.9 | 581.7 | 1449.4 | 1652.9 | 975.1 | 1056.1 | 428.8 |
| 90 | 603.0 | 1089.4 | 846.8 | 568.4 | 898.5 | 971.4 | 1600.4 | 939.7 | 346.8 |

Note: In the tables above, "-" indicates a value lower than the detection limit.

As can be seen from the results of the tables above, the concentration of plasma anesthetic (propofol) peaked immediately before the termination of the anesthetic (30 minutes after the beginning of administration) in each group and gradually decreased after that. While no significant differences were observed among the groups, the lactated-Ringer's solution group tended to show a higher plasma anesthetic concentration than the other groups at the termination of the anesthetic, and 60 and 90 minutes after the termination of the anesthetic.
This observation suggests that the prompt arousal reaction observed in the bicarbonated-Ringer's solution group resulted partly from the faster decrease in the plasma anesthetic concentration in this group than in the lactated-Ringer's solution group.

### Example 5: Effect of pH on the binding of propofol to human serum albumin

### [Method]

A 50mM phosphate buffer was prepared and the pH was adjusted to 7.0, 7.2, 7.4, 7.6 and 7.8. Human serum albumin (HAB, hereinafter) was added to each solution to a concentration of 40 mg/mL.
Subsequently, propofol dissolved in methanol was added to each phosphate buffered solution to a final concentration of 5 µg/mL. The solutions were immediately stirred and equilibrated at 37°C for 30 minutes. After equilibration, 1 mL of each solution was centrifuged. Free propofol (protein-unbound form of propofol) in the filtrate was quantified by HPLC.

### [Results]

The test was conducted twice and the proportion of free propofol concentration with respect to the total propofol concentration in each phosphate buffer solution was determined (in average). The results are shown in Table 10.
The proportion of free propofol increased as the pH of the solution was decreased, suggesting that the protein binding of propofol varies as changes of the blood pH: more propofol exists in its free form at acidic pHs. That more propofol exists in its free form at acidic pHs implies the possibility that the anesthetic effect of propofol is enhanced in acidosis as compared to in the normal state of the blood even if the total propofol concentration is constant.

**(Table 10)**

| | | | | | |
|---|---|---|---|---|---|
| pH | 7.0 | 7.2 | 7.4 | 7.6 | 7.8 |
| Free form (%) | 2.27 | 2.21 | 2.02 | 1.88 | 1.44 |

### Example 6: Relationship between acidosis and awakening time in a rat model of streptozotocin (STZ)-induced diabetic ketoacidosis (part 1)

### [Method]

STZ was dissolved in 0.1M citrate buffer to form an aqueous STZ solution. This solution was administered to rats from the tail vein at a dose of 100 mg/kg/mL. After 48 hours, the blood gas was measured to confirm the onset of acidosis. Subsequently, a test infusion fluid (a Ringer's solution prepared according to Japanese Pharmacopoeia) was injected via a catheter placed in the central vein at a rate of 20 mL/kg/hr for 90 minutes. At the same time, propofol (1% Diprivan injection) was administered for 90 minutes. The time required for awakening from anesthesia was measured.

### [Results]

The relationship between the blood pH and the time it took before emergence is shown in Fig. 1.
As can be seen from the results shown in the figure, the individuals that had had severe acidosis before the administration of anesthetic tended to take longer to awaken from anesthesia, indicating the inverse relationship between the awakening time and the severity of acidosis. This indicates that acidosis is involved in the arousal from anesthesia and suggests the possibility that fast correction of acidosis will significantly affect the awakening from anesthesia.

### Example 7: Awakening time in a rat model of STZ-induced diabetic ketoacidosis (part 2) - Comparison of the present invention with acetated-Ringer's solution -

### [Method]

STZ was dissolved in 0.1M citrate buffer to form an aqueous STZ solution. This solution was administered to rats from the tail vein at a dose of 100 mg/kg/mL. After 48 hours, the blood gas was measured to confirm the onset of acidosis. The animals were divided into three groups of 7 or 8 animals (= n) having substantially equal blood pHs. The three groups received a test infusion fluid (bicarbonated-Ringer's solution of the present invention), an acetated-Ringer's solution and a Ringer's solution prepared according to Japanese Pharmacopoeia (Ringer's solution, hereinafter), respectively. The solutions were administered via a catheter placed in the central vein at a rate of 20 mL/kg/hr for 90 minutes. At the same time, propofol (1% Diprivan injection) was administered for 90 minutes. The time required for awakening from anesthesia was measured.

### [Results and discussion]

The time it took for each group before awakening was as follows:
Bicarbonated-Ringer group: 33.7 ± 21.5 min
Acetated-Ringer group: 63.4 ± 21.0 min
Ringer group: 53.6 ± 21.5 min
The results show that the bicarbonated-Ringer group (present invention) took the least time to awaken from anesthesia in all of the three groups tested. The acetated-Ringer group took as much time as the Ringer group. The awakening time of the bicarbonated-Ringer group (present invention) was significantly shorter than that of the acetated-Ringer group (p < 0.05).
These results can be interpreted as follows: the alkalization effect of sodium acetate provided by the acetated-Ringer's solution is decreased because the metabolism of ketone bodies produced in large quantities in diabetic ketoacidosis interferes with the metabolism of acetic acid. Thus, the ability of the acetated-Ringer's solution to correct acidosis is decreased, affecting the awakening time.
In contrast, it has been demonstrated that the bicarbonated-Ringer's solution of the present invention can quickly correct acidosis and can thereby accelerate awakening from anesthesia.

### INDUSTRIAL APPLICABILITY

According to the present invention, a bicarbonate ion-containing Ringer's solution is used in perioperative (intraoperative) patients as a substitute for the blood to replace extracellular fluid. The Ringer's solution of the present invention serves to correct acidosis and thereby facilitate prompt awakening of patients from anesthesia. Also, the infusion fluid is stable and enhances the recovery of the systemic metabolism by facilitating prompt awakening. It also enables fast recovery of spontaneous respiration and allows tissues and organs to resume their normal function quickly. As a result, increased risk of complication caused by the delayed action of biological defense or decreased immune activity can be avoided.

In addition, the care required by patients in ICU because of delayed awakening from anesthesia can be reduced and, as a result, the workload of medical staff can be decreased. In this regard, the present invention is of significant medical importance.

## Claims

1. A composition comprising a bicarbonate ion, for use in a method of anesthesia arousal for a post operative patient.

2. The composition according to claim 1,
wherein the bicarbonate ion is contained as an electrolyte.

3. The composition according to claim 1 or 2, comprising sodium bicarbonate as a major component that serves as a source of the bicarbonate ion, along with each or a combination of another electrolyte, glucose and an amino acid.

4. The composition according to claim 1, 2 or 3, provided in a form of Ringer's solution.

## Patentansprüche

1. Zusammensetzung, die ein Bicarbonation umfasst, zur Verwendung in einem Verfahren zum Aufwecken eines postoperativen Patienten nach einer Narkose.

2. Zusammensetzung nach Anspruch 1, wobei das Bicarbonation als Elektrolyt enthalten sind.

3. Zusammensetzung nach Anspruch 1 oder 2, die Natriumbicarbonat als Hauptbestandteil, der als Quelle des Bicarbonations dient, zusammen mit einem weiteren Elektrolyt, Glucose oder einer Aminosäure oder einer Kombination davon umfasst.

4. Zusammensetzung nach Anspruch 1, 2 oder 3, die in Form einer Ringer-Lösung bereitgestellt ist.

## Revendications

1. Composition comprenant un ion bicarbonate, pour l'utilisation dans un procédé de réveil après une anesthésie à destination d'un patient post-opératoire.

2. Composition selon la revendication 1, dans laquelle l'ion bicarbonate est contenu sous la forme d'un électrolyte.

3. Composition selon la revendication 1 ou 2, comprenant du bicarbonate de sodium en tant que composant majeur qui sert de source de l'ion bicarbonate, conjointement avec chacun ou une combinaison d'un autre électrolyte, de glucose et d'un acide aminé.

4. Composition selon la revendication 1, 2 ou 3, se présentant sous la forme d'une solution de Ringer.
